# EUROPEAN PATENT APPLICATION

(11) **EP 4 732 746 A1**
(43) Date of publication of application: **29.04.2026**
(21) Application number: 25197216.2
(22) Date of filing: 21.08.2025
(51) Int. Cl.: A61B 1/00, A61B 1/018, A61B 1/307

(54) **HAND SURGICAL INSTRUMENT AND BRIDGE FOR A HAND SURGICAL INSTRUMENT**

(30) Priority: 22.10.2024 DE 102024130759
(71) Applicant: Olympus Winter & Ibe GmbH, 22045 Hamburg (DE)
(72) Inventor: Rühs, Andreas, 22926 Ahrensburg (DE); Schröder, Bastian, 22589 Hamburg (DE); Merkel, Alexander, 22607 Hamburg (DE)
(74) Representative: Hoener, Matthias

(57) **Abstract**

The invention provides a bridge for a hand surgical instrument, with which all working instruments and the optics can be inserted into and removed from the hand surgical instrument in a simple and clear manner. This is achieved in that a bridge (12) for a hand surgical instrument (10) has a main body (20) in which a channel-like bore (19) for an optical system and at least one working channel (26, 28) for receiving at least one working instrument are arranged. A plane (24) can be laid through a longitudinal axis (18), which divides the space around the bridge (12) into an upper half-space (25) and a lower half-space (26). The at least one working channel (26, 28) extends from the main body (20) in the direction of the upper half-space (25).

## Description

The invention relates to a bridge for a hand surgical instrument according to claim 1. The invention further relates to a hand surgical instrument according to claim 10.

For urological applications, hand-held surgical instruments such as endoscopes, cystoscopes, resectoscopes and the like are known to be used. The instruments are guided with a tube-like shaft through an opening in the body of the person to be treated and placed in the position intended for the application. Depending on the application or instrument, a wide variety of working instruments are guided through the shaft, such as forceps, scissors, clamps or, as in the case of high-frequency surgery, electrical electrodes. In addition, together with the aforementioned working instruments, an optical system is guided through the shaft into the body to provide visual control of the treatment.

Outside the body, namely at the proximal end of the shaft, there is usually an instrument bridge. Such a bridge is used to attach the various working instruments and the optics that are to be guided into the shaft. For this purpose, it is intended that bores, valves or other closure or connection means are arranged on a main body of the bridge. It is also conceivable that the main body has connections for, for example, a fluid outlet or supply. Further embodiments are known in which additional electrical or optical instruments can be connected to this main body of the bridge.

In the known hand surgical instruments, the connections and the working channels for receiving working instruments are arranged on a lower side or a lower half of the bridge, so that the openings of the connections or the working channels point downwards or away from the bridge. In practice, it is often the case that the hand surgical instruments inserted into the patient's body are oriented in such a way that the openings of the connections or working channels also point downwards or towards the patient's body. This makes it difficult to insert the working instruments into the working channels or to connect any tubes etc. to the connections. The surgeon performing the treatment must more or less connect the instruments to the connections without being able to see them directly. This means that the surgeon either has to change position or that connecting the instruments takes an unnecessarily long time. As soon as all the instruments or other tubes etc. are connected to the main body of the bridge, the situation becomes very confusing for the surgeon, as he cannot see at a glance which working channels or connections are occupied and which are not, or which instruments the channels and connections are occupied with.

Based on this, the problem of the present invention is to create a bridge for a hand surgical instrument with which all working instruments and the optics can be inserted into and removed from the bridge of the hand surgical instrument in a simple and clear manner.

A bridge for solving this problem has the features of claim 1. Accordingly, it is provided that a bridge for a hand surgical instrument, which may, for example, be an endoscope, a cystoscope or a resectoscope, has a main body in which a channel-like bore for an optical system and at least one working channel for receiving at least one working instrument are arranged. A distal end of this bridge can be coupled to a shaft for the optics and the at least one working instrument. A proximal end of the bridge can be connected to a telescope of the optics. A longitudinal axis of the bridge can be drawn through the bore of the main body or the bridge. A plane can be placed on or through this longitudinal axis, which divides the space around the bridge into two half-spaces, namely an upper half-space and a lower half-space. The upper half-space comprises the side of the bridge, which is generally referred to as the upper side, and in particular comprises the connection for the telescope. The lower half-space generally comprises the connections or working channels. Preferably, holes or the connection for the optics are associated with the upper half-space and the connections or working channels for receiving further working instruments are associated with the lower half-space. An essential feature of the invention is that the at least one working channel extends from the main body in the direction of the upper half-space. This orientation of the at least one working channel for receiving a working instrument makes it easier for the surgeon to insert working instruments into the working channel during treatment of the patient, since these point in the direction of the surgeon and not downwards or in the direction of the patient. Similarly, this orientation of the working channels makes the working environment clearer for the surgeon, as it is immediately apparent which instruments are in which connections or channels.

It is also preferable for the at least one working channel to extend from the main body into the upper half-space. This extended version of the working channel moves the individual openings or connections away from each other, so that the surgeon has more space available to insert the instruments into the working channels.

In particular, it is conceivable that the at least one working channel is arranged in the lower half-space of the bridge on the main body and extends out of the lower half-space in the direction of the upper half-space or into the upper half-space. This embodiment of the bridge is particularly advantageous, as the working channels are easily accessible for fitting instruments and provide a high degree of clarity.

Furthermore, it may preferably be provided that the at least one working channel is arranged in the upper half-space of the bridge on the main body and extends into the upper half-space. Similarly, it is conceivable that the at least one working channel is arranged exactly in the plane or at least partially intersects the plane and extends into the upper half-space. Depending on the requirements of the treatment to be carried out, different embodiments of the bridge may prove advantageous. In particular, the relative arrangement of the optics and the working instruments in the instrument may require a corresponding arrangement of the working channels relative to the bore.

A further advantageous embodiment of the invention may provide for a first working channel to be arranged on one side of the bore and a second working channel on the other side of the bore. In this case, the two working channels are arranged symmetrically or parallel offset relative to the plane. If a left and a right side are defined with respect to the longitudinal axis of the bore or the instrument, the first working channel would be assigned to the left side and the second working channel to the right side. This relative arrangement of the working channels means that both can be fitted with an instrument in a very simple and clear manner, without the two instruments interfering with each other.

It is also preferably provided that the at least one working channel forms an acute angle with the bore, with the angle opening in a proximal direction. Due to the diverging course of the bore and the at least one working channel, sufficient space is created at the corresponding openings or access points of the bore and the working channel to serve the openings with the instruments.

It is also conceivable that the at least one working channel has a radius such that one end of the working channel extends in the direction of the upper half-space. Due to this slight curvature of the at least one working channel, it is guided from its orientation in the main body upwards or into the upper half-space in such a way that the instrument can still be moved in the working channel in a simple manner.

Finally, it is conceivable that the at least one working channel has a valve through which the channel can be closed. This valve can be used to prevent liquids or other media from unintentionally entering the instrument during treatment and thus leading to contamination, or to prevent a medium from escaping from the instrument in an uncontrolled manner during treatment. This valve can then be designed as a manually operated valve. It is also conceivable that the proximal opening of the working channel can be closed by a plug or other cap.

A hand surgical instrument, in particular an endoscope, a cystoscope, a resectoscope or the like, for solving the said problem has the features of claim 10. Accordingly, it is provided that the hand surgical instrument has a bridge according to at least one of claims 1 to 9.

A preferred embodiment of the invention is explained in more detail below with reference to the drawing. This shows:
- Fig. 1: a schematic representation of a hand surgical instrument,
- Fig. 2: a perspective view of a bridge, and
- Fig. 3: a side view of a bridge.

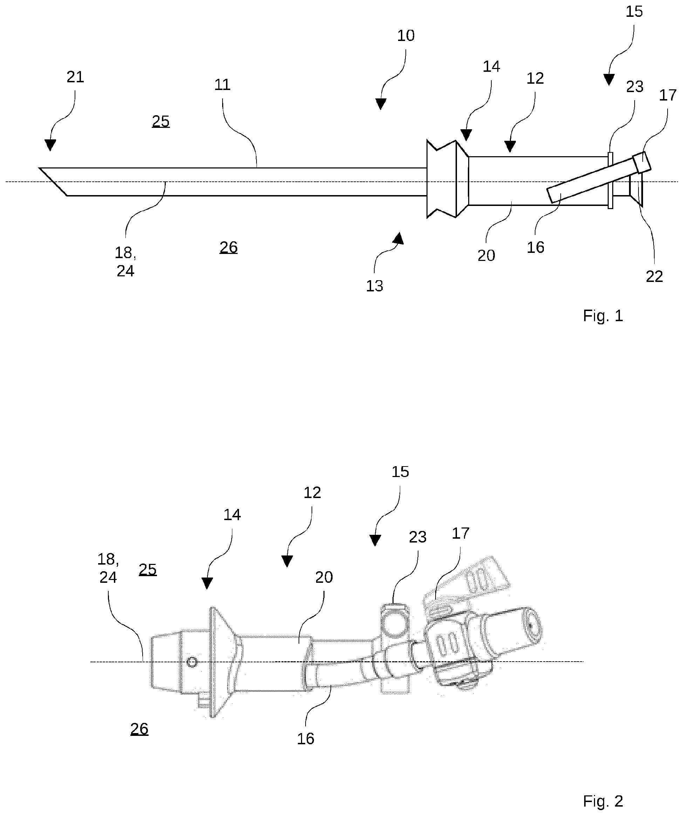

Fig. 1 shows a highly schematized hand surgical instrument 10. This instrument 10 may, for example, be a cystoscope, a resectoscope or a similar instrument for minimally invasive treatment of a patient. The hand surgical instrument 10 described here essentially consists of a tube-like shaft 11 and a bridge 12. To treat the patient, the instrument 10 is guided with a distal end 21 of the shaft 11 into a body opening of the patient. The bridge 12 is located outside the body. Various working instruments can be inserted into the body through the shaft 11 via the bridge 12. In addition, the treatment can be observed or controlled via the bridge 12 and the shaft 11 using appropriate optics.

The shaft 11 can be coupled with a proximal end 13 to a distal end 14 of the bridge 12. For example, a screw lock, a click lock, a bayonet lock or a clamping ring can be used for this purpose. A telescope 22 with an optical system can be arranged at a proximal end 15 of the bridge 12. The bridge 12 essentially consists of a main body 20. In Fig. 1, a working channel 16 is assigned to this main body 20 in a highly schematized manner. This working channel 16 is tubular and can have a valve 17 at one free end.

Within the bridge 12 there are at least two channel-like passages which run at least substantially parallel to a longitudinal axis 18 of the hand surgical instrument 10 or the shaft 11, namely a bore 19 and at least one channel, not shown, which opens into the working channel 16. The bore 19 and the channel also run largely parallel to each other and to the longitudinal axis 18.

The working channel 16 is an extension of the channel. Various working instruments, such as wires, probes, clamps, catheters, stents, electrodes, flexible instruments or the like, can be inserted into the hand surgical instrument 10 through this tube-like working channel 16 when the valve 17 is open. The working instrument, which is not shown, is guided through the main body 20 or the bridge 12 and through the shaft 11 to a distal end 21 of the shaft 11. The treatment of the patient takes place in front of the distal end 21 of the shaft.

The hole 19 is used to accommodate an optic not shown. This optic can, for example, be a rod lens system not shown. This rod lens system extends from the proximal end 15 to the distal end 14 of the bridge 12 and is guided further through the entire shaft 11 to the distal end 21 of the shaft 11, where it is directed towards the area to be treated. At the proximal end 15 of the bridge 12, the optics or the rod lens system can be connected to the telescope 22. The telescope 22 and/or the rod lens system can be attached to an optical plate 23 of the bridge 12. An eyepiece or a camera can be attached to the telescope 22 so that the surgeon can view the area to be operated on via the rod lens system. As an alternative to the rod lens system, a fiber optic can also be used as an imaging device.

A plane 24 can be laid through the longitudinal axis 18, which passes through the hole 19, which projects vertically from the drawing plane. This imaginary plane 24 divides the space around the bridge 12 into two half-spaces, namely an upper half-space 25 and a lower half-space 26. The upper half-space 25 represents the area that is usually referred to as the top of the instrument 10 or the bridge 12. This is where the optics are arranged on the telescope 22, at least for the most part. The lower half-space 26, on the other hand, is also understood to be the underside of the instrument 10 or the bridge 12. In the embodiment example shown here, the working channel 16 is led out of the main body 20. Locking means at the distal end 14 of the bridge 12 may also be associated with the lower half-space 26.

Fig. 2 clearly shows that the working channel 16 leads out of the main body 20 in the lower half-space 26 and then has a slight curvature or radius so that the working channel 16 leads into the upper half-space 25. An opening 27 or the valve 17 connected to the opening 27 is located completely or almost completely in the upper half-space 25. It is also conceivable that the opening 27 is located in the lower half-space 26, but is oriented in the direction of the upper half-space 25. The working channel 16 thus breaks through the plane 24 and points upwards or into the upper half-space 25 and thus in the direction of the operator. This orientation makes it particularly easy for the surgeon to insert instruments not shown into the working channel 16. Similarly, hoses or other connections, for example, can be coupled to the valve 17 in a very simple and clear manner. By moving the working channel 16 upwards in this way, the entire surgical situation is very clear for the surgeon. This is because no channels, tubes, instruments or the like are covered by other instruments etc. or guided in such a way that it is not directly visible how the various openings of the instrument 10 are fitted.

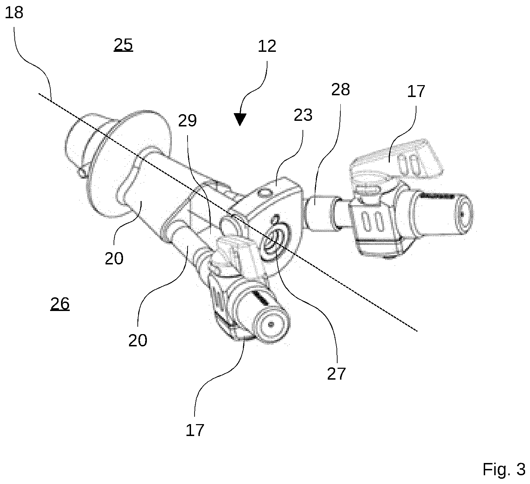

The perspective view of Fig. 3 shows that in the embodiment example shown here, two working channels 16, 28 are assigned to the bridge 12 or the main body 20. These two working channels 16, 28 are arranged symmetrically with respect to the longitudinal axis 18 and are oriented in the same way with respect to the plane 24, i.e. both point into the upper half-space 25. It can also be seen from Fig. 3 that the two working channels 16 and 28 form an acute angle 29 with the longitudinal axis 18, this angle 29 opening towards the proximal end 15 of the bridge 12. This divergent relative arrangement of the two working channels 16 and 28 further increases the space available to the surgeon for using the instrument 10, which further simplifies or improves the manageability of the instrument 10.

Similarly, embodiments are conceivable in which the bridge 12 has only a single working channel 16 or more than two working channels 16. Similarly, it is conceivable that the working channels 16, 28 are led out of the main body 20 in the plane 24 or in the upper half-space 25.

### List of Reference Numerals:

- 10: Instrument
- 11: Shaft
- 12: Bridge
- 13: Proximal end
- 14: Distal end
- 15: Proximal end
- 16: Working channel
- 17: Valve
- 18: Longitudinal axis
- 19: Bore
- 20: Main body
- 21: Distal end
- 22: Telescope
- 23: Optical plate
- 24: Plane
- 25: Upper half space
- 26: Lower half-space
- 27: Opening
- 28: Working channel
- 29: Angel

## Claims

1. Bridge (12) for a hand surgical instrument (10), in particular an endoscope, a cystoscope or a resectoscope, having a main body (20) which has a channel-like bore (19) for an optical system and at least one working channel (16, 28) for receiving at least one working instrument, wherein a distal end (14) of the bridge (12) can be coupled to a shaft (11) for the optical system and the at least one working instrument (16, 28) and a proximal end (15) can be coupled to a telescope (22), a longitudinal axis (18) of the bridge (12) extending axially through the bore (19) and a plane (24) passing through this longitudinal axis (18) and defining an upper half-space (25) and a lower half-space (26) around the bridge (12), **characterized in that** the at least one working channel (16, 28) extends from the main body (20) in the direction of the upper half-space (25).

2. Bridge (12) for a hand surgical instrument (10) according to claim 1, **characterized in that** the at least one working channel (16, 28) extends from the main body (20) into the upper half-space (25).

3. Bridge (12) for a hand surgical instrument (10) according to claim 1 or 2, **characterized in that** the at least one working channel (16, 28) is arranged in the lower half-space (26) of the bridge (12) on the main body (20) and extends out of the lower half-space (26) in the direction of the upper half-space (25) or into the upper half-space (25).

4. Bridge (12) for a hand surgical instrument (10) according to one of the preceding claims, **characterized in that** the at least one working channel (16, 28) is arranged in the upper half-space (25) of the bridge (12) on the main body (20) and extends into the upper half-space (25).

5. Bridge (12) for a hand surgical instrument (10) according to one of the preceding claims, **characterized in that** the at least one working channel (16, 28) is arranged exactly in the plane (24) or at least partially intersects the plane (24) and extends into the upper half-space (25).

6. Bridge (12) for a hand surgical instrument (10) according to one of the preceding claims, **characterized by** a first working channel (16) and a second working channel (28) arranged on different sides of the bore (19).

7. Bridge (12) for a hand surgical instrument (10) according to one of the preceding claims, **characterized in that** the at least one working channel (16, 28) includes an acute angle (29) with the bore (19), wherein the angle (29) opens in the proximal direction.

8. Bridge (12) for a hand surgical instrument (10) according to one of the preceding claims, **characterized in that** the at least one working channel (16, 28) has a radius such that one end of the working channel (16, 28) extends in the direction of the upper half-space (25).

9. Bridge (12) for a hand surgical instrument (10) according to one of the preceding claims, **characterized in that** the at least one working channel (16, 28) has a valve (17).

10. Hand surgical instrument (10), in particular an endoscope, a cystoscope, a resectoscope or the like, with a bridge (12) according to any one of claims 1 to 9.
